# EUROPÄISCHE PATENTSCHRIFT

(11) **EP 0 880 945 B1**
(45) Veröffentlichungstag und Bekanntmachung des Hinweises auf die Patenterteilung: **26.04.2006**
(21) Anmeldenummer: 98108718.2
(22) Anmeldetag: 13.05.1998
(51) Int. Cl.: A61C 19/00

(54) **Lichtpolymerisationsgerät**
Photopolymerization device
Dispositif de photopolymérisation

(30) Priorität: 26.05.1997 DE 29709228 U
(43) Veröffentlichungstag der Anmeldung: 02.12.1998
(73) Patentinhaber: 3M ESPE AG, 82229 Seefeld (DE)
(72) Erfinder: Herold, Wolf-Dietrich, Dr., 82229 Seefeld (DE); Kürschner, Ralf, 82131 Gauting (DE); Koran, Peter, Dr., 82362 Weilheim (DE)

(56) Entgegenhaltungen:
- EP-A- 0 599 224
- EP-A- 0 678 282
- EP-A- 0 755 662
- EP-A- 0 772 247
- EP-A- 0 780 103
- US-A- 5 420 768

## Beschreibung

In der Dentaltechnik ist eine Vielzahl von Kunststoffen, sogenannten Composites, bekannt, die aufgrund eines methacrylatischen Härtungsmechanismus unter Lichteinstrahlung polymerisieren. Als wesentlichen Photoinitiator enthalten diese Materialien Campherchinon oder Phosphinoxid, die im blauen Spektralbereich breitbandig, mit einem Absorptionsmaximum bei etwa 472 nm bzw. 430 nm, absorbieren.

Die Polymerisationsreaktion erfordert in einer sehr dünnen Schicht je nach Materialfarbe eine Lichtintensität von mindestens 1 bis 5 mW/cm². Bei der Polymerisation von Zahnfüllungen oder Zahnersatzteilen ist in der Praxis zur Erzielung eines ausreichenden Polymerisationsgrades und einer ausreichenden Polymerisationstiefe innerhalb einer vertretbaren Zeitspanne eine Lichtintensität von mindestens 250 mW/cm² erforderlich. Auf dem Markt befindliche dentale Polymerisationsgeräte emittieren Licht mit einer Intensität von etwa 400 bis 500 mW/cm², gelegentlich bis zu 700 mW/cm².

Bekannt sind Tischgeräte, bei denen das Licht im Gerät erzeugt und fokussiert und mittels eines flexiblen Lichtleiters von typisch 1,5 bis 2 m Länge an den Behandlungsort im Mund des Patienten geleitet wird. Abgesehen von den erheblichen Lichtverlusten an seinen Eintritts- und Austrittsflächen ist ein derartiger Lichtleiter mit seinem üblichen Durchmesser von etwa 10 bis 15 mm verhältnismäßig steif und unhandlich.

Bei anderen bekannten Geräten (vgl. DE-A-3 840 984) wird das Licht in einem pistolenartig gestalteten Handteil erzeugt und fokussiert und mittels eines starren Faser- oder Quarz-Lichtleiterstabes an den Behandlumgsort gebracht. Ein Hauptnachteil dieser Geräte besteht in der erheblichen Erwärmung des Handgerätes und, da es in der Nähe des Behandlungsortes gehalten wird, der bestrahlten Stelle selbst. Im übrigen wird auch bei diesem Gerätetyp das zur Stromversorgung des Handteils erforderliche Netzkabel als störend empfunden.

Bekannt sind auch akkubetriebene Geräte (vgl. DE-A-4 211 230), die zwar unabhängig vom Netz bzw. einem Netzteil sind, wegen der erforderlichen hohen elektrischen Leistungen jedoch verhältnismäßig große und schwere Akkumulatoren benötigen und daher entsprechend unhandlich sind.

Die bekannten Lichtpolymerisationsgeräte arbeiten häufig mit Wolfram-Halogen-Lampen, die in einem verhältnismäßig breiten Spektralbereich emittieren und daher zum größten Teil Energie als Wärme und Licht im roten und grünen Wellenlängenbereich abgeben. Nur ca. zwei Prozent der aufgenommenen Leistung wird im Spektralbereich von etwa 400 bis 515 nm emittiert, der für die erwähnten Composite-Materialien mit Campherchinon oder Phosphinoxid als Photoinitiator brauchbar ist.

Bei den herkömmlich verwendeten Lichtquellen besteht ferner die Schwierigkeit, daß ihre Lichtleistung über die Lebensdauer in einer für den Benutzer nicht ohne weiteres feststellbaren Weise nachläßt, so daß die Qualität der Polymerisation im Lauf der Zeit abnimmt.

Ein weiterer Nachteil besteht darin, daß optische Komponenten wie Linsen und Reflektoren nötig sind, um die Wendel des Brenners auf den Eingang des Lichtleiters in geeignetem Maß abzubilden, so daß er voll ausgeleuchtet wird, jedoch kein Licht verlorengeht. Zudem werden Filter benötigt, die die Wärmestrahlung absorbieren und das Halogenlicht auf den gewünschten Spektralbereich reduzieren. Auch diese Komponenten können durch Alterung oder Defekte die Lichtleistung reduzieren, so daß eine sichere Polymerisation nicht mehr möglich ist.

Ein weiterer Nachteil der bekannten Geräte, insbesondere der Handgeräte, besteht darin, daß die zur Wärmeabfuhr notwendige Luftströmung gleichzeitig eine Verteilung von Bakterien bewirkt, wobei die Geräte außerdem aufgrund ihrer zu Belüftungszwecken notwendigerweise offenen Bauform und ihrer Größe schlecht sterilisiert oder desinfiziert werden können.

Aus EP-A-0 780 103 ist ein Lichtpolymerisationsgerät mit den im ersten Teil des Anspruchs 1 sowie in den Ansprüchen 2, 3, 5 und 8 angegebenen Merkmalen bekannt.

EP-A-0 755 662 offenbart ein Lichtpolymerisationsgerät, das als Lichtquelle eine ebenfalls im blauen Spektralbereich emittierende, jedoch aus Elementen der II. und IV. Hauptgruppe des Periodensystems gebildete Laserdiode verwendet.

Der Erfindung liegt die generelle Aufgabe zugrunde, Nachteile, wie sie bei vergleichbaren Geräten nach dem Stand der Technik auftreten, mindestens teilweise zu vermeiden. Eine speziellere Aufgabe der Erfindung besteht darin, ein Gerät zur Lichtpolymerisation von Kunststoffen, insbesondere von mit Campherchinon oder Phosphinoxid als Photoinitiator arbeitenden Dentalmaterialien anzugeben, das bei möglichst hohem Wirkungsgrad Licht in dem nutzbaren blauen Spektralbereich erzeugt und eine Regelung der Lichtleistung gestattet.

Die erfindungsgemäße Lösung dieser Aufgabe ist im Anspruch 1 gekennzeichnet.

Die Verwendung des beanspruchten Festkörperstrahlers in einem insbesondere dentalen Polymerisationsgerät ergibt folgende Vorteile:
(1) Da die Lichtemission auf einen bestimmten Wellenlängenbereich, hier den blauen Bereich, begrenzt ist, wird keine zusätzliche Wärme erzeugt. Dies bedeutet, daß das Gerät ohne Lüfter auskommt und daher als geschlossenes, gekapseltes und insgesamt sterilisierbares Gerät herstellbar ist.
(2) Die Vermeidung jeglicher Wärmeerzeugung ist ferner für den Polymerisationsvorgang selbst von Vorteil, da der durch Erwärmungs- und Abkühlungsvorgänge hervorgerufene Schrumpf des Kunststoffmaterials mit der sich daraus ergebenden Gefahr von Randspalten vermieden wird.
(3) Bei Festkörperstrahlern verändert sich ferner die Lichtleistung im Laufe der Zeit. Da sich die Leistung einfach durch Nachregeln des Diodenstroms korrigieren läßt, kann dieser Effekt kompensiert werden. Da Laserdioden zwei entgegengesetzte Lichtstrahlen aussenden, bieten sie grundsätzlich die Möglichkeit, einen dieser Strahlen als Nutzstrahl zur Polymerisation und den anderen als Referenzstrahl zur Messung der Regelungsgröße zu verwenden.
(4) Die Tatsache, daß der Festkörperstrahler ausschließlich in dem schmalen nutzbaren Spektralbereich emittiert, bedeutet, daß er bei ausreichender Strahlungsintensität mit geringer Leistungsaufnahme auskommt. Das Gerät kann daher ohne weiteres von einer eingebauten Batterie gespeist werden, so daß die bei den bekannten Geräten erforderlichen Lichtleiter oder Stromversorgungskabel entfallen.
(5) Die geringe Größe des Festkörperstrahlers in Verbindung mit dem Fehlen jeglicher Wärmeentwicklung gestattet es schließlich, das Gerät klein, leicht und handlich zu bauen.
(6) Da die Lichterzeugung praktisch trägheitslos geschieht, ist auch gepulster Betrieb möglich, wobei sehr hohe Intensitäten für kurze Zeit erreicht werden. Dies führt zu einer höheren Transparenz des zu polymerisierenden Materials, da durch den hohen Photonenstrom alle Absorptionsniveaus des Materials besetzt werden können.
(7) Durch die divergente, elliptische Abstrahlungscharakteristik der Laserdioden kann im Gegensatz zu herkömmlichen Lasern auf eine Optik zur optimalen Ausleuchtung des Lichtleiters verzichtet werden.
(8) Die Intensität des Referenzstrahls, der auf der Rückseite der Laserdiode emittiert wird, wird mittels einer Photodiode gemessen und dient über eine Regelelektronik zur Regelung der Lichtleistung des Nutzstrahls.

Gemäß Anspruch 2 handelt es sich bei dem Halbleiter insbesondere um einen solchen auf der Basis von Galliumnitrid mit geeigneten ternären Zusätzen.

Wird der Festkörperstrahler gemäß der Ausgestaltung der Erfindung nach Anspruch 3 im LED-Modus betrieben, so ergibt sich der Vorteil einer flächigen Abstrahlung bei nicht allzu schmaler Bandbreite. Beide Eigenschaften sind für die Anwendung im Dentalbereich besonders günstig, weil eine größere Abstrahlungsfläche den üblichen Dimensionen der zu bestrahlenden Zahnflächen näher kommt und die größere Spektralbreite der Absorptionsbandbreite der die Polymerisation initiierenden Moleküle besser entspricht, woraus eine effiziente Polymerisation resultiert.

Ein besonders intensiver Lichtstrahl ergibt sich dagegen beim Betrieb des Festkörperstrahlers im Lasermodus gemäß Anspruch 4.

Die geringe Größe des Festkörperstrahlers gestattet es ferner, ihn gemäß Anspruch 5 unmittelbar an der auf den Behandlungsort richtbaren Spitze des Polymerisationsgerätes anzuordnen. Bei dieser Ausgestaltung ergibt sich der Vorteil besonders geringer Strahlungsverluste.

In der Variante nach Anspruch 6 kann der Festkörperstrahler am Lichteintrittsende eines auf den Behandlungsort richtbaren Lichtleiters angeordnet sein. Auch bei dieser Ausführungsform, bei der sich der Strahler im Innern des Gerätes befindet, läßt sich wegen der fehlenden Wärmeerzeugung ein äußerst kleines und handliches Gerät erzielen.

In der Weiterbildung nach Anspruch 7 enthält das Gerät auch eine Batterie oder einen Akkumulator zur Versorgung des Festkörperstrahlers.

Anspruch 8 betrifft den gepulsten Betrieb, der eine wesentlich höhere Strahlintensität zuläßt, was zu einer höheren Eindringtiefe des Lichtstrahls in das zu polymerisierende Material führt.

Nach Anspruch 9 lassen sich Diode und Lichtleiter derart fest zueinander orientieren, daß durch die Verwendung einer an den Strahlquerschnitt geometrisch angepaßten, gemäß Anspruch 10 vorzugsweise elliptischen, Eintrittsapertur des Lichtleiters der Nutzstrahl vollständig abgebildet werden kann.

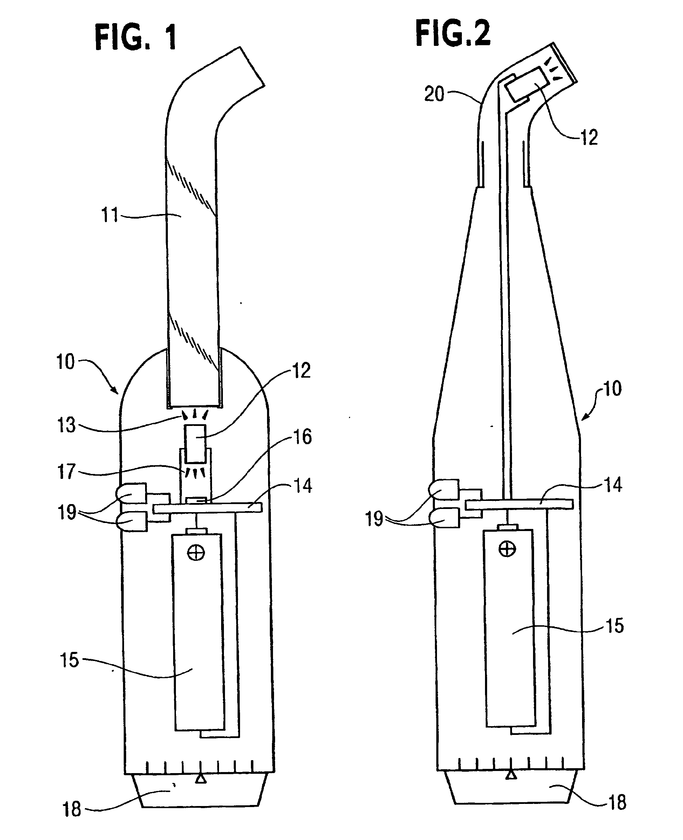

Im folgenden werden zwei Ausführungsbeispiele des erfindungsgemäßen Lichtpolymerisationsgerätes anhand der Figuren 1 und 2 der beigefügten Zeichnung näher erläutert.

Das Gerät nach Figur 1 besteht aus einem Handteil 10, in dessen vorderes Ende ein Lichtleiterstab 11 eingesteckt ist. Zur praktischeren Handhabung ist Lichtleiterstab 11 ist an seinem Lichtaustrittsende gekrümmt.

Das Lichteintrittsende des Lichtleiterstabes 11 empfängt den von einem Festkörperstrahler 12 abgegebenen Nutzstrahl 13. Bei dem Festkörperstrahler handelt es sich um eine im blauen Spektralbereich emittierende, vorzugsweise gepulst betriebene Leuchtdiode, die aus Elementen der III. und V. Hauptgruppe des Periodensystems, vorzugsweise Galliumnitrid aufgebaut ist. Zur Anpassung an den elliptischen Querschnitt des Nutzstrahls 13 hat der Lichtleiterstab 11 mindestens an seinem Lichteintrittsende ebenfalls elliptischen Querschnitt.

Das Handteil 10 enthält ferner eine Leiterplatte 14 mit einer integrierten Schaltung zur Ansteuerung des Festkörperstrahlers 12 und eine Batterie 15 zur Energieversorgung.

An der Leiterplatte 14 ist ein mit der integrierten Schaltung verbundener Sensor 16 angeordnet, der den an der Rückseite der Leuchtdiode 12 austretenden Strahl 17 als Referenzstrahl zur Regelung der Lichtleistung der Leuchtdiode und damit des Nutzstrahls 13 empfängt.

An seinem vom Lichtleiterstab 11 abgewandten Ende ist das Handteil 10 mit einem als Drehknopf ausgebildeten Zeiteinsteller 18 versehen. Zur optischen Anzeige des Betriebszustandes sind ferner seitlich am Handteil 10 zwei Leuchtdioden 19 geringer Strahlungsleistung angeordnet.

Der Lichtleiterstab 11 kann in bestimmten Ausführungsformen dem Handteil 10 gegenüber drehbar sein, so daß sich das Lichtaustrittsende in die jeweils günstigste Stellung relativ zum Bestrahlungsort drehen läßt, ohne daß die Stellung des Handteils 10 und damit des Zeiteinstellers 18 und der Leuchtdioden 19 verändert werden muß.

Das Gerät nach Figur 2 unterscheidet sich von dem nach Figur 1 dadurch, daß der Festkörperstrahler 12 unmittelbar an der auf den Behandlungsort richtbaren Spitze des Gerätes angeordnet ist. Die den Strahler 12 enthaltende Spitze 20 des Gerätes ist ähnlich wie das Lichtaustrittsende des Lichtleiterstabes 11 nach Figur 1 gekrümmt und gegenüber dem Handteil 10 des Gerätes drehbar, so daß sich die jeweils günstigste Stellung relativ zum Behandlungsort ohne Stellungsänderung des Handteils selbst erreichen läßt.

## Patentansprüche

1. Gerät zur Polymerisation von Kunststoffen, insbesondere von Photoinitiatoren enthaltenden Dentalmaterialien, mittels Licht im blauen Spektralbereich, wobei die Lichtquelle ein Festkörperstrahler (12) auf der Basis eines aus Elementen der III. und V. Hauptgruppe des Periodensystems gebildeten Halbleiters ist, **dadurch gekennzeichnet, daß** der Festkörperstrahler (12) eine Laserdiode ist, deren nicht zur Polymerisation verwendeter, dem Nutzstrahl (13) entgegengesetzter rückwärtiger Laserstrahl als Referenzsignal (17) zur Intensitätsregelung des Nutzstrahls verwendet wird.

2. Gerät nach Anspruch 1, wobei der Halbleiter aus Galliumnitrid besteht.

3. Gerät nach Anspruch 1 oder 2, wobei der Strahler (12) im LED-Modus arbeitet.

4. Gerät nach Anspruch 1 oder 2, wobei der Strahler (12) im Lasermodus arbeitet.

5. Gerät nach einem der Ansprüche 1 bis 4, wobei der Festkörperstrahler (12) unmittelbar an der auf den Behandlungsort richtbaren Spitze (20) des Gerätes angeordnet ist.

6. Gerät nach einem der Ansprüche 1 bis 4, wobei der Festkörperstrahler (12) am Lichteintrittsende eines auf den Behandlungsort richtbaren Lichtleiters (11) angeordnet ist.

7. Gerät nach einem der vorhergehenden Ansprüche, wobei es eine Batterie (15) oder einen Akkumulator zur Energieversorgung des Festkörperstrahlers (12) enthält.

8. Gerät nach einem der vorhergehenden Ansprüche, wobei die Festkörperstrahler (12) gepulst betrieben wird.

9. Gerät nach Anspruch 6 oder nach Anspruch 7 oder 8, soweit auf Anspruch 6 rückbezogen, wobei Lichtleiter (11) und Festkörperstrahler (12) zueinander ortsfest und orientiert sind und daß die Eingangsapertur des Lichtleiters in Form und Abmessung dem Querschnitt des Laserstrahls entspricht.

10. Gerät nach Anspruch 9, wobei die Eingangsapertur die Form einer Ellipse besitzt.

## Claims

1. Device for polymerization of plastics, in particular of dental materials containing photoinitiators, by means of light in the blue spectral region, the light source being a solid state emitter (12) based on a semiconductor formed from elements from main groups III and V of the periodic table, **characterized in that** the solid state emitter (12) is a laser diode whose back laser beam not used for polymerization and opposing the useful beam (13) is used as reference signal (17) for regulating the intensity of the useful beam.

2. Device according to Claim 1, in which the semiconductor consists of gallium nitride.

3. Device according to Claim 1 or 2, in which the emitter (12) operates in the LED mode.

4. Device according to Claim 1 or 2, in which the emitter (12) operates in the laser mode.

5. Device according to one of Claims 1 to 4, in which the solid state emitter (12) is arranged directly at the tip (20) of the device which can be aimed at the treatment site.

6. Device according to one of Claims 1 to 4, in which the solid state emitter (12) is arranged at the light entry end of a light guide (11) which can be aimed at the treatment site.

7. Device according to one of the preceding claims, in which it includes a battery (15) or a rechargeable battery for supplying energy to the solid state emitter (12).

8. Device according to one of the preceding claims, in which the solid state emitter (12) is operated in a pulsed fashion.

9. Device according to Claim 6 or according to Claim 7 or 8, to the extent referred back to Claim 6, in which the light guide (11) and solid state emitter (12) are fixed and orientated relative to one another, and the input aperture of the light guide corresponds in shape and dimension to the cross section of the laser beam.

10. Device according to Claim 9, in which the input aperture has the shape of an ellipse.

## Revendications

1. Appareil pour la polymérisation de matériaux synthétiques, en particulier de matériaux dentaires contenant des photoinitiateurs, à l'aide d'une lumière dans le domaine spectral bleu, dans lequel la source lumineuse est un émetteur de rayonnement (12) sur la base d'un semi-conducteur formé d'éléments des groupes principaux III et V du système périodique, **caractérisé en ce que** l'émetteur de rayonnement (12) est une diode laser dont le rayon laser qui n'est pas utilisé pour la polymérisation et qui part vers l'arrière dans le sens opposé du rayon utile (13) est utilisé comme signal de référence (17) pour la régulation de l'intensité du rayon utile.

2. Appareil selon la revendication 1, dans lequel le semi-conducteur est constitué de nitrure de gallium.

3. Appareil selon la revendication 1 ou 2, dans lequel l'émetteur (12) fonctionne en mode DEL.

4. Appareil selon la revendication 1 ou 2, dans lequel l'émetteur (12) fonctionne en mode laser.

5. Appareil selon l'une quelconque des revendications 1 à 4, dans lequel l'émetteur de rayonnement (12) est aménagé directement sur la pointe de l'appareil orientable (20) vers la zone de traitement.

6. Appareil selon l'une quelconque des revendications 1 à 4, dans lequel l'émetteur de rayonnement (12) est aménagé à l'extrémité de l'entrée de la lumière d'un conducteur optique (11) orientable vers la zone de traitement.

7. Appareil selon l'une quelconque des revendications précédentes, lequel appareil comporte une batterie (15) ou un accumulateur pour l'alimentation en énergie de l'émetteur de rayonnement (12).

8. Appareil selon l'une quelconque des revendications précédentes, dans lequel l'émetteur de rayonnement (12) peut fonctionner par impulsions.

9. Appareil selon la revendication 6, ou selon la revendication 7 ou 8 pour autant que l'on se réfère à la revendication 6, dans lequel le conducteur optique (11) et l'émetteur de rayonnement (12) sont solidaires l'un de l'autre et orientés, et l'ouverture d'entrée du conducteur optique correspond en termes de forme et de dimension à la section du rayon laser.

10. Appareil selon la revendication 9, dans lequel l'ouverture d'entrée présente la forme d'une ellipse.
